# EUROPEAN PATENT SPECIFICATION

(11) **EP 3 351 171 B1**
(45) Date of publication and mention of the grant of the patent: **25.12.2019**
(21) Application number: 18153123.7
(22) Date of filing: 24.01.2018
(51) Int. Cl.: A61B 5/097, A61B 5/08, A62B 23/00, A61M 16/10, A62B 9/06, B01D 46/00

(54) **MOUTHPIECE, METHOD FOR MANUFACTURING THE SAME, AND BREATH TEST DEVICE**
MUNDSTÜCK, VERFAHREN ZUR HERSTELLUNG DAVON UND ATEMTESTVORRICHTUNG
EMBOUT BUCCAL, SON PROCÉDÉ DE FABRICATION ET DISPOSITIF DE TEST RESPIRATOIRE

(30) Priority: 24.01.2017 JP 2017010254
(43) Date of publication of application: 25.07.2018
(73) Proprietor: Japan Precision Instruments Inc., Shibukawa-shi Gumma 377-0293 (JP)
(72) Inventor: YOSHIDA, Akira, Gunma, 377-0293 (JP)
(74) Representative: Betten & Resch

(56) References cited:
- EP-A2- 2 745 776
- JP-A- H1 133 015
- JP-A- 2012 022 182
- US-A1- 2013 133 663

## Description

### Technical field

The present invention relates to a mouthpiece, a method for manufacturing the same, and a breath test device.

### Description of related art

Conventionally, as a mouthpiece with a filter, for example, those described in Patent Documents 1 to 3 are known.

US 2013/133663 A1 discloses a respiratory device filter which comprises a housing having a mouthpiece and a body. The housing defines an inner sidewall and a conical filter is disposed within the housing. The conical filter includes a first end that defines an apex and a second end that defines an opening. An apex retention member extends from the inner sidewall and is secured to the apex of the conical filter.

### Prior art document

### Patent document

[Patent Document 1] Published Japanese Translation of PCT International Application No. 2016-500151
[Patent Document 2] Japanese Unexamined Patent Application Publication No. 2013-55981
[Patent Document 3] Japanese Unexamined Patent Publication No. Hei 11-33015

### Summary of the invention

### Problem to be solved by the invention

Generally, since the mouthpiece is used by being held in the mouth, it is often provided as a disposable product in consideration of a hygiene aspect of a user. Therefore, it is desirable that the mouthpiece be provided as inexpensively as possible.

However, the mouthpiece described in each of the above Patent Documents 1 to 3 has a more complicated constitution because, the filter is attached inside of the mouthpiece, and a cost becomes high.

An object of the present invention is to provide a technique capable of providing a mouthpiece with a filter at a lower cost than before.

### Means for solving the problem

### (First aspect)

A first aspect of the present invention provides a mouthpiece, including:
a tubular mouthpiece main body configured to form a flow path for flowing a breathing gas;
a filter placed on the flow path; and
a tubular insertion member inserted into the mouthpiece main body, in order to hold the filter in the inside of the mouthpiece main body and having a length not to protrude from the mouthpiece main body;
wherein the filter has a first filter portion corresponding to a cross-sectional shape of the flow path and a second filter portion folded-back from an outer circumferential edge of the first filter portion, and the second filter portion is sandwiched between an inner circumferential surface of the mouthpiece main body and an outer circumferential surface of the insertion member.

### (Second aspect)

A second aspect of the present invention provides the mouthpiece of the first aspect, wherein a folded-back dimension of the second filter portion is 2 mm or more.

### (Third aspect)

A third aspect of the present invention provides the mouthpiece of the first or second aspect, wherein a curved tapered is provided at one end portion of the mouthpiece main body.

### (Fourth aspect)

A fourth aspect of the present invention provides a breath test device, including:
the mouthpiece of any one of the first to third aspects, and
a member to which the mouthpiece is detachably attached,
wherein a predetermined component contained in an exhaled breath supplied through the mouthpiece is tested.

### (Fifth aspect)

A fifth aspect of the present invention provides a method for manufacturing a mouthpiece, including:
preparing a tubular mouthpiece main body configured to form a flow path for flowing a breathing gas, a filter placed on the flow path, and a tubular insertion member inserted into the mouthpiece main body in order to hold the filter in the inside of the mouthpiece main body and having a length not to protrude from the mouthpiece main body; and forming a first filter portion corresponding to a cross-sectional shape of the flow path by
covering one end portion of the insertion member with the filter, and
sandwiching a second filter portion being a folded-back portion of the filter between an inner circumferential surface of the mouthpiece main body and an outer circumferential surface of the insertion member, by folding-back a part of the filter and inserting the insertion member into the mouthpiece main body.

### Advantage of the invention

According to the present invention, a mouthpiece with a filter can be provided at a lower cost than before.

### Brief description of the drawings

FIG. 1 is a cross-sectional view showing a constitutional example of a mouthpiece according to an embodiment of the present invention.
FIG. 2 (A) is a view of the mouthpiece shown in FIG. 1 viewed from a left direction, and FIG. 2 (B) is a view thereof viewed from a right direction.
FIG. 3 is an exploded perspective view showing a constitutional example of the mouthpiece according to an embodiment of the present invention.
FIG. 4 is a view for explaining an assembly jig used for manufacturing the mouthpiece according to an embodiment of the present invention and a method for manufacturing a mouthpiece using the same.
FIG. 5 is a front view showing a relative positional relationship between a filter and an insertion member.
FIG. 6 is a side view showing a constitutional example of a breath test device according to an embodiment of the present invention.
FIG. 7 is a plan view showing a constitutional example of the breath test device according to an embodiment of the present invention.
FIG. 8 (A) is a view of the breath test device shown in FIG. 6, viewed from a left direction, and FIG. 8 (B) is a view thereof viewed from a right direction.

### Detailed description of the invention

Embodiments of the present invention will be described hereafter in detail, with reference to the drawings. In the embodiment of the present invention, explanation will be given in the following order.
1. Constitution of a mouth piece
2. Method for manufacturing a mouthpiece
3. How to use a mouthpiece
4. Constitution of a breath test device
5. How to use a breath test device
6. Effect of the embodiment
7. Modified example

### <1. Constitution of a mouthpiece>

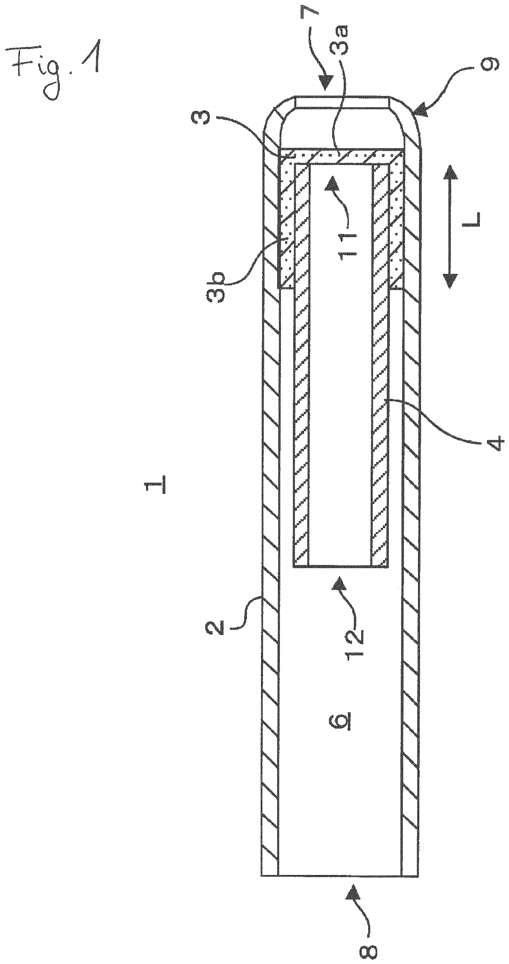

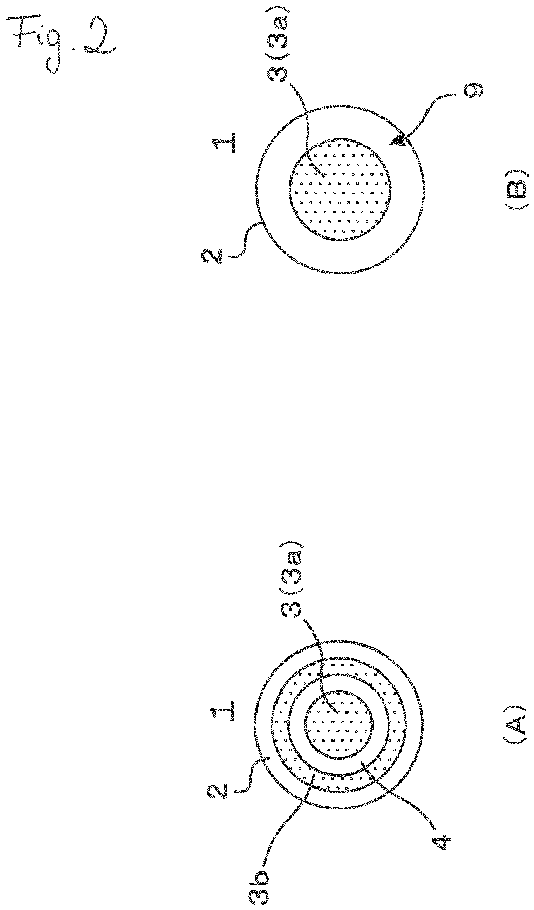

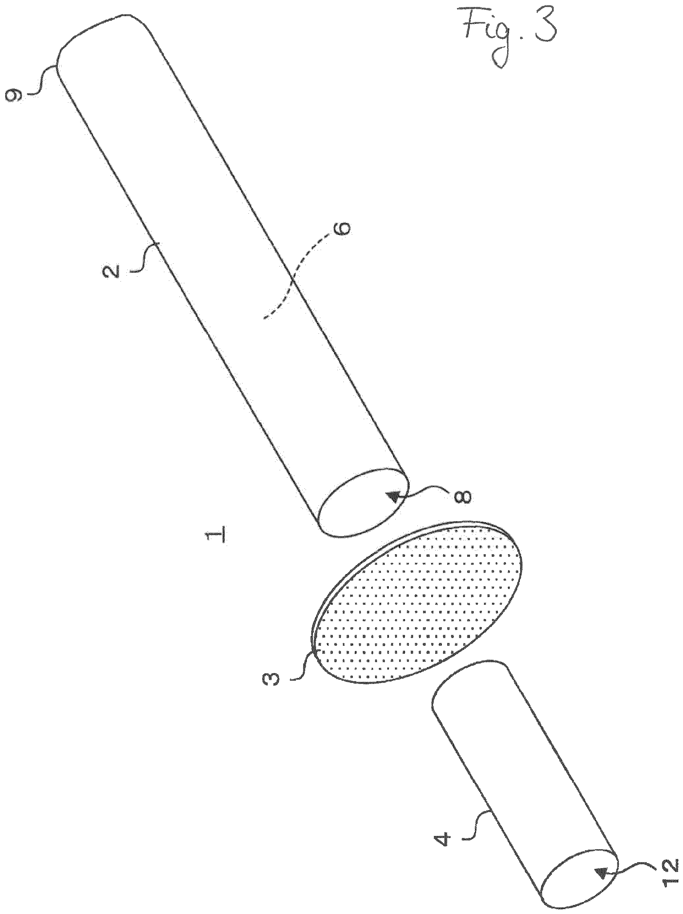

FIG. 1 is a cross-sectional view showing a constitutional example of a mouthpiece according to an embodiment of the present invention, and FIG. 2 (A) is a view of the mouthpiece shown in FIG. 1 viewed from a left direction, and FIG. 2 (B) is a view thereof viewed from a right direction. Further, FIG. 3 is an exploded perspective view showing a constitutional example of the mouthpiece according to an embodiment of the present invention.

A mouthpiece 1 shown in the figure includes a tubular mouthpiece main body 2, a filter 3, and a tubular insertion member 4.

### (Mouthpiece main body)

The mouthpiece main body 2 forms a flow path 6 for flowing a breathing gas. The "breathing gas" described here means "exhaled breath and / or inhaled breath". The mouthpiece main body 2 is made of, for example, a resin, preferably a thermoplastic resin, more preferably polypropylene. Further, the mouthpiece main body 2 is preferably made of a transparent or translucent material so that positions and conditions of the filter 3 and the insertion member 4 placed inside of the mouthpiece main body 2 can be visually confirmed from outside.

The mouthpiece main body 2 is formed into a tubular (cylindrical) shape having a circular cross section. Therefore, the mouthpiece 1 has a straw shape as a whole. The "straw shape" described here means the tubular shape with a central axis extending straight (linearly). A thickness of the mouthpiece main body 2 is preferably set to 0.2 mm or more and 1.0 mm or less. A length of the mouthpiece main body 2 is preferably set to 5 cm or more and 10 cm or less. However, the thickness and the length of the mouthpiece main body 2 can be appropriately set or changed respectively according to a use purpose of the mouthpiece 1. The length of the mouthpiece main body 2 is a dimension in a central axis direction of the mouthpiece main body 2.

An opening 7 is formed at one end portion of the mouthpiece main body 2 in the central axis direction and an opening 8 is formed at the other end portion thereof. The openings 7 and 8 are each formed into a circular shape. One end portion of the mouthpiece main body 2 in a central axis direction is a curved taper portion 9 rounded inward. In this curved taper portion 9, inner and outer diameters of the mouthpiece main body 2 are gradually decreased toward the opening 7. In contrast, on the other end side of the mouthpiece main body 2, the inner diameter and the outer diameter of the mouthpiece main body 2 are uniformly the same diameter toward the opening 8. Therefore, the diameter of the opening 8 is larger than the diameter of the opening 7.

### (Filter)

The filter 3 is provided in the mouthpiece 1 in order to collect dust, viruses, bacteria, saliva and the like contained in the breathing gas and prevent infection. The filter 3 is preferably made of a polypropylene nonwoven fabric. The filter 3 is preferably made of a material having a performance of BFE (bacterial filtration efficiency) of 75% or more and PFE (fine particle filtration efficiency) of 93% or more. The filter 3 has a moderate thickness. The performance of the filter 3 is determined by a fineness of mesh and a thickness dimension of a material (nonwoven fabric or the like) constituting the filter 3, and therefore it is preferable to constitute the filter 3 using a material that satisfies a desired performance.

The filter 3 is placed on the flow path 6 formed by the mouthpiece main body 2. The filter 3 is placed in the vicinity of the opening 7 in the central axis direction of the mouthpiece main body 2. The filter 3 integrally has a first filter portion 3a corresponding to the cross-sectional shape of the flow path 6, and a second filter portion 3b which is folded-back from the outer circumferential edge of the first filter portion 3a. The cross-sectional shape of the flow path 6 refers to the shape of the flow path 6 when the mouthpiece main body 2 is sectioned in a direction orthogonal to the central axis direction thereof. In this embodiment, the mouthpiece main body 2 is formed into a cylindrical shape.

Therefore, the cross-sectional shape of the flow path 6 is circular, and correspondingly, the shape of the first filter portion 3a is also circular. The shape of the first filter portion 3a described here refers to the shape of the first filter portion 3a when viewing the filter 3 from the center axis direction of the mouthpiece main body 2.

The second filter portion 3b is folded-back at a right angle from the outer circumferential edge of the first filter portion 3a. The second filter portion 3b is sandwiched between the inner circumference surface of the mouthpiece main body 2 and the outer circumferential surface of the insertion member 4. "Sandwiching" means to support the filter portion by interposing it between the mouthpiece main body and the insertion member. A difference between the inner diameter of the mouthpiece main body 2 and the outer diameter of the insertion member 4 is set to be smaller than the thickness dimension of the filter 3, so that an appropriate compressive force is applied to the filter 3 (the second filter portion 3b) in a gap sandwiched between the mouthpiece man body 2 and the insertion member 4. For example, when it is assumed that the thickness dimension of the filter 3 is 0.5 mm, a gap dimension generated due to the difference between the inner diameter of the mouthpiece main body 2 and the outer diameter of the insertion member 4 is set to 0.4 mm or less.

The folded-back dimension L of the second filter portion 3b is preferably 2 mm or more, more preferably 3.5 mm or more, and further preferably 5 mm or more. However, if the folded-back dimension L is too long, a wasteful portion which does not substantially function as the filter 3 is increased by the folded-back dimension L. Further, there is a possibility that an assembling property at the time of manufacturing the mouthpiece 1 is deteriorated. Therefore, the folded-back dimension L of the second filter portion 3b is preferably 20 mm or less, more preferably 15 mm or less, further preferably 10 mm or less.

### (Insertion member)

The insertion member 4 is a member to be inserted into the mouthpiece main body 2 in order to hold the filter 3 in the inside of the mouthpiece main body 2. The insertion member 4 is placed concentrically with the mouthpiece main body 2 in the inside (in-tube) of the mouthpiece main body 2. The insertion member 4 is made of, for example, a resin, preferably a thermoplastic resin, more preferably polypropylene which is the same material as the mouthpiece main body 2. The insertion member 4 is formed into a tubular (cylindrical) shape having a circular cross section, similarly to the mouthpiece main body 2. The thickness of the insertion member 4 is preferably set to 0.2 mm or more and 1.0 mm or less, similarly to the mouthpiece main body 2. The length of the insertion member 4 is set to be shorter than the length of the mouthpiece main body 2, in order to prevent the insertion member 4 from protruding from the mouthpiece main body 2. For example, if the length of the mouthpiece main body 2 is 5 cm or more and 10 cm or less, the length of the insertion member 4 is set to 3 cm or more and less than 5 cm. However, the length of the insertion member 4 can be appropriately set or changed according to the length of the mouthpiece main body 2. The length of the insertion member 4 is the dimension in the central axis direction of the insertion member 4.

An opening 11 is formed at one end of the insertion member 4 in the central axis direction, and an opening 12 is formed at the other end thereof. The opening 11 of the insertion member 4 is covered with the filter 3 (first filter portion 3a). For convenience of explanation, different reference numerals are given to the opening 11 and the opening 12 here, but the insertion member 4 has a symmetrical structure in the central axis direction thereof, and therefore the positional relationship between the opening 11 and the opening 12 may be reversed. The inner diameter and the outer diameter of the insertion member 4 are uniformly the same diameter over an entire central axis direction. Therefore, the diameter of the opening 11 and the diameter of the opening 12 are set to the same diameter. The outer diameter of the insertion member 4 is set to be smaller than the inner diameter of the mouthpiece main body 2. Further, the outer diameter of the insertion member 4 is preferably set to be larger than a diameter of the opening 7 of the mouthpiece main body 2.

### <2. Method for manufacturing a mouthpiece>

Subsequently, a method for manufacturing a mouthpiece according to an embodiment of the present invention will be described.

First, the mouthpiece main body 2, the filter 3 and the insertion member 4 which are components of the mouthpiece 1, are prepared.

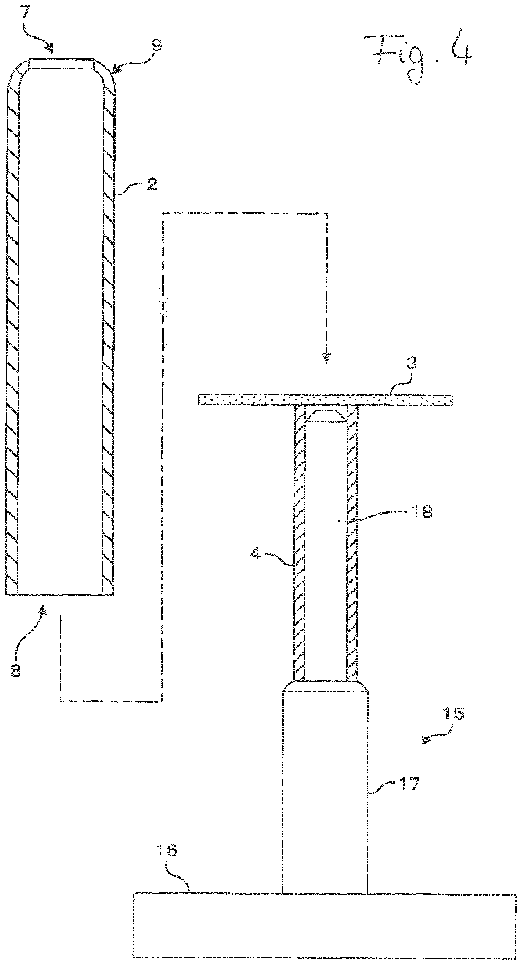

Next, as shown in FIG. 4, the insertion member 4 and the filter 3 are set in the assembly jig 15. The assembly jig 15 includes a base portion 16, a large-diameter shaft portion 17 standing from the base portion 16, and a small-diameter shaft portion 18 standing from an upper end of the large-diameter shaft portion 17. The base portion 16 is formed into a flat plate shape. The large-diameter shaft portion 17 and the small-diameter shaft portion 18 are placed coaxially with each other. The outer diameter of the small-diameter shaft portion 18 is set to be smaller than the inner diameter of the insertion member 4. The length of the small-diameter shaft portion 18 is set to be shorter than the length of the insertion member 4. The outer diameter of the large-diameter shaft portion 17 is set to be larger than the outer diameter of the insertion member 4 and smaller than the inner diameter of the mouthpiece main body 2. The combined length of the large-diameter shaft portion 17 and the small-diameter shaft portion 18 is set to be shorter than the length of the mouthpiece main body 2.

When the insertion member 4 and the filter 3 are set in the assembly jig 15 having the above constitution, first, by inserting the insertion member 4 into the small-diameter shaft portion 18, the lower end portion of the insertion member 4 is butted against the upper end portion of the large-diameter shaft portion 17. Thereby, the insertion member 4 is vertically supported by the small-diameter shaft portion 18.

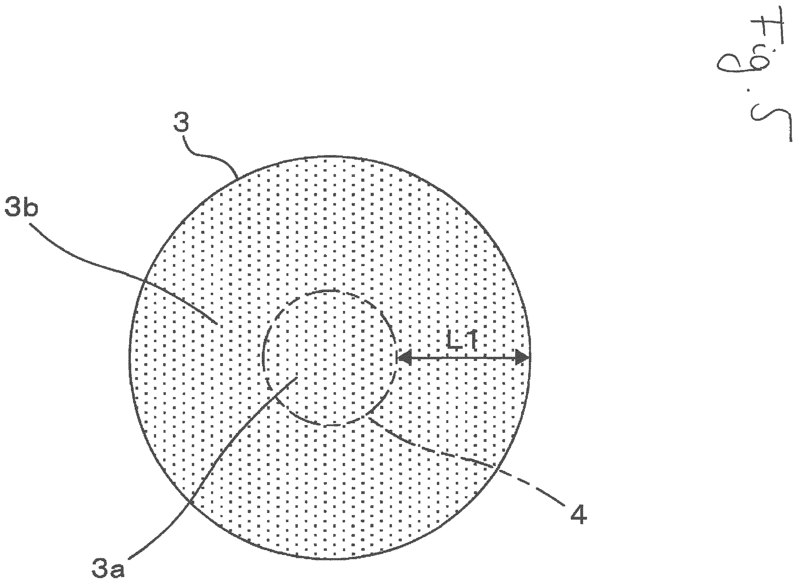

Next, the filter 3 is put on the upper end portion of the insertion member 4. The filter 3 is formed into a circular shape in front view (FIG. 3) corresponding to the cross-sectional shape of the mouthpiece main body 2 and the insertion member 4. At this time, as shown in FIG. 5, the central portion of the filter 3 is aligned with the insertion member 4 so that the filter 3 and the insertion member 4 are placed concentrically. At this time, fine irregularities which exhibit an anti-slip effect may be formed on the opening end of the insertion member 4, so that the position of the filter 3 placed on the insertion member 4 is not easily deviated. Thereby, it is possible to suppress positional deviation of the filter 3 placed on the upper end portion of the insertion member 4, and suppress falling caused thereby, and the like.

Next, the mouthpiece main body 2 is placed right above the insertion member 4, with the opening 8 facing downward, and thereafter the mouthpiece main body 2 is pushed from above the filter 3. At this time, the outer portion of the filter 3 is pushed by the mouthpiece main body 2 and folded-back. Further, the insertion member 4 is inserted (press-fitted) into the mouthpiece main body 2. As a result, the folded-back portion (the second filter portion 3b) of the filter 3 is sandwiched between the inner circumferential surface of the mouthpiece main body 2 and the outer circumferential surface of the insertion member 4. The filter 3 may be folded-back by an operator's hand before the mouthpiece main body 2 is pushed from above the filter 3. Further, by forming fine irregularities on the opening end of the insertion member 4 for preventing slippage, the positional deviation of the filter 3 at the time of pushing the mouthpiece main body 2, can be suppressed.

Further, by securing a moderate overlap length L1 (FIG. 5) on the outside of the filter 3 when the insertion member 4 is covered with the filter 3, the folded-back portion (second filter portion 3b) of the filter 3 can be secured over an entire circumference of the insertion member 4 in the circumferential direction, even if the position of the filter 3 is deviated in the process of pushing the mouthpiece main body 2. The overlap length L1 of the filter 3 corresponds to the difference between the outer diameter of the insertion member 4 and the outer diameter of the filter 3 before the outside of the filter 3 is folded-back. Therefore, when the outside of the filter 3 is folded-back to provide the second filter portion 3b, the folded-back dimension L (FIG. 1) of the second filter portion 3b has a dimension equivalent to the overlap length LI, on the assumption that there is no positional deviation of the filter 3.

When the mouthpiece main body 2 is pushed as described above, the mouthpiece main body 2 may be pushed until the opening 8-side end portion of the mouthpiece main body 2 is butted against the base portion 16, or the mouthpiece main body 2 may be pushed until the filter 3 placed on the insertion member 4 is butted against the curved taper portion 9 of the mouthpiece main body 2. In the latter case, when the insertion member 4 is inserted into the mouthpiece main body 2, the curved taper portion 9 of the mouthpiece main body 2 functions as a stopper. Therefore, the mouthpiece main body 2 and the insertion member 4 can be relatively aligned with each other using the stopper function of the curved taper portion 9.

As described above, the mouthpiece 1 shown in FIG. 1 is obtained.

### <3. How to use the mouthpiece>

The mouthpiece 1 according to the embodiment of the present invention is used by the user by holding it in the mouth. At that time, the user holds the opening 7 side end portion of the mouthpiece main body 2 in the mouth. At this time, by forming the curved taper portion 9 on the mouthpiece main body 2, mouthfeel becomes soft due to the roundness of the curved taper portion 9. Further, splashes of saliva do not easily enter the mouthpiece 1 when the user breathes, while holding the mouthpiece 1 in the mouth.

When the user breathes while holding the mouthpiece 1 in the mouth as described above, an exhaled breath and an inhaled breath flow through the mouthpiece main body 2 as follows. First, the exhaled breath flows from the opening 7 of the mouthpiece main body 2 toward the flow path 6, and flows out of the opening 8 through the filter 3 placed on the flow path 6. At this time, bacteria, viruses, etc. contained in the exhaled breath are collected by the filter 3. On the other hand, the inhaled breath flows into the flow path 6 from the opening 8 of the mouthpiece main body 2 and flows out from the opening 7 through the filter 3 placed on the flow path 6. At this time, bacteria, viruses and the like contained in the inhaled breath are collected by the filter 3. Accordingly, by using a new mouthpiece 1 for each user, the effect of preventing infection can be obtained.

### <4. Constitution of a breath test device>

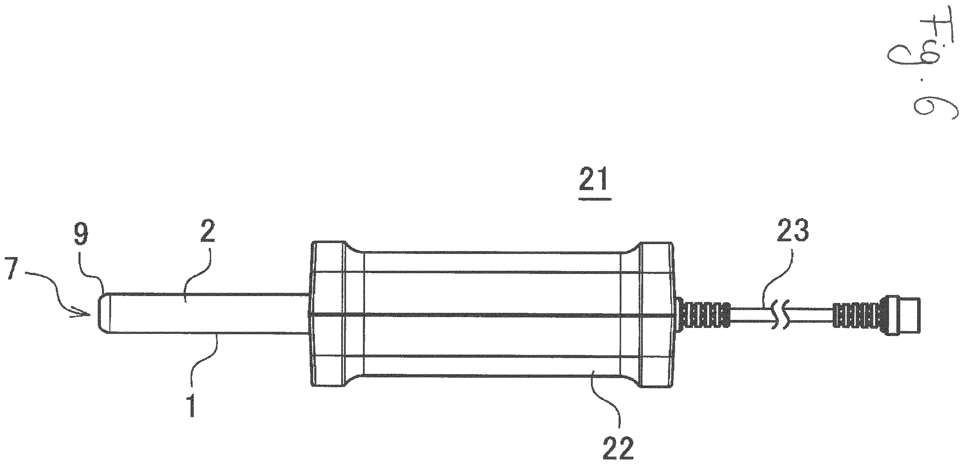

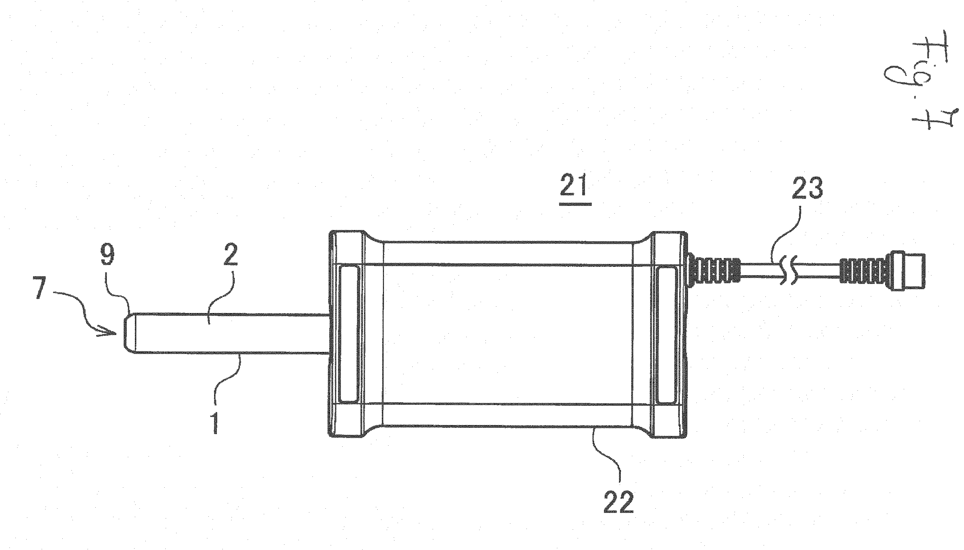

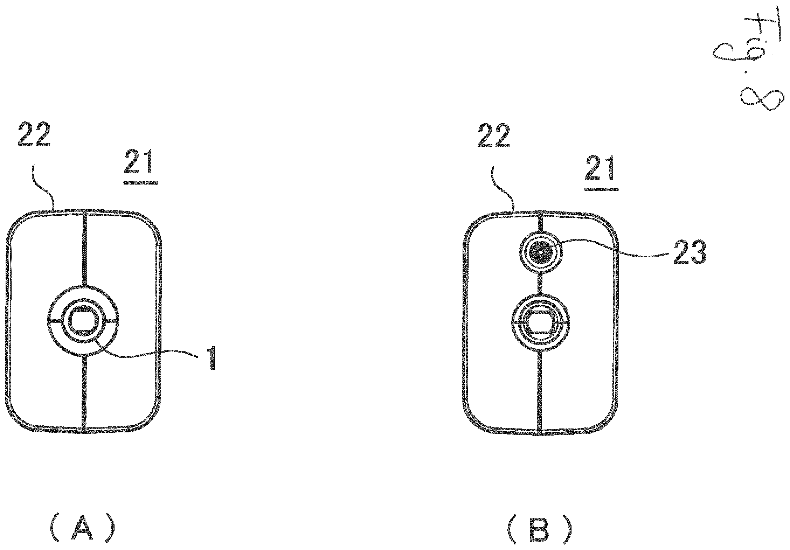

FIG. 6 is a side view showing a constitutional example of a breath test device according to an embodiment of the present invention, and FIG. 7 is a plan view thereof. Further, FIG. 8 (A) is a view of the breath test device shown in FIG. 6, viewed from a left direction, and FIG. 8 (B) is a view thereof viewed from a right direction.

The breath test device 21 shown in the figure includes a case 22, a mouthpiece 1, and a cable 23. A passage (not shown) which serves as a passageway for the exhaled breath or the inhaled breath of the user using the mouthpiece 1, and a sensor (not shown) for inspecting a predetermined component contained in the exhaled breath passing through this passage, are provided inside of the case 22. This sensor inspects (detects) a predetermined gas concentration contained in the exhaled breath, for example, as a component in the exhaled breath supplied into the case 22 through the mouthpiece 1. As an example of the predetermined gas concentration, the concentration of carbon dioxide can be given. In this case, the sensor may be constituted using a light emitting unit for emitting infrared rays, and a light receiving unit for receiving the infrared rays emitted from the light emitting unit.

The mouthpiece 1 is attached to the case 22. The case 22 is provided with a tubular portion (not shown). The tubular portion communicates with the passage in the case 22. Communication means to be connected spatially. The mouthpiece 1 is detachably attached to the tubular portion of the case 22. Specifically, when the mouthpiece 1 is attached to the case 22, the opening 8 of the mouthpiece main body 2 is fitted into the tubular portion of the case 22. Thereby, the opening 7 and the curved taper portion 9 of the mouthpiece main body 2 are placed on the side farther from the case 22. Therefore, the user who uses the breath test device 21 can easily hold the opening 7 side end portion of the mouthpiece 1 in the mouth. On the other hand, when the mouthpiece 1 is detached from the case 22, the mouthpiece 1 (mouthpiece main body 2) is pulled in a direction away from the case 22. Thereby, the mouthpiece 1 attached to the case 22 can be exchanged. Here, the case 22 is given as an example of a member to which the mouthpiece 1 is detachably attached, but the mouthpiece 1 may also be attached to other members.

The cable 23 is a cable for electrically connecting the breath test device 21 and a measurement device (not shown). A length of the cable 23 is set to an appropriate length as necessary. The cable 23 is used for supplying electric power to drive the sensor from the measurement device to the breath test device 21, or exchanging a control signal for controlling the driving of the sensor between the measurement device and the breath test device 21, or outputting a result detected by the sensor as an electric signal. The breath test device 21 and the measurement device are separately constituted here, but the present invention is not limited thereto, and the breath test device 21 and the measurement device may be integrally constituted.

### <5. How to use a breath test device>

Subsequently, a method for using the breath test device 21 according to the embodiment of the present invention will be described.

First, the breath test device 21 with a mouthpiece 1 is provided in the hand of a testee (user) who will undergo a breath test. Next, the testee is asked to have the opening 7 side of the mouthpiece 1 held in his/her mouth. Next, a tester lets the testee breathe naturally, and performs a breath test using a sensor in this situation. In this breath test, the concentration of carbon dioxide in the exhaled breath is tested as an example of a predetermined component contained in the exhaled breath of the testee. The concentration of carbon dioxide in the exhaled breath is expressed by EtCO₂. EtCO₂ is a term meaning an end tidal carbon dioxide concentration (end tidal CO₂). For measuring the carbon dioxide concentration (EtCO₂), for example, it is preferable to use a sensor utilizing mid-infrared rays having a wavelength of 3.75 µm or more and 4.25 µm or less.

Actually when the testee breathes with the mouthpiece 1 held in his/her mouth, the exhaled breath and the inhaled breath alternately flow through the flow path 6 in the mouthpiece main body 2 in accordance with a breathing of the testee. Further, the exhaled breath and the inhaled breath also flow alternately in the passage inside of the case 22 of the breath test device 21. At this time, a movement direction of the exhaled breath flowing through the passage when the testee breathes out, and a movement direction of the inhaled breath flowing through the passage when the testee breathes in, are opposite to each other. Namely, when the testee breathes out, the exhaled breath flows from the mouthpiece 1 toward the passage inside of the case 22, and when the testee breathes in, the inhaled breath flows from the passage inside of the case 22 toward the mouthpiece 1.

Under such a circumstance, the gas concentration detection sensor emits infrared light from the light emitting unit, and by receiving this infrared ray by the light receiving unit, the concentration of carbon dioxide in the exhaled breath flowing in the passage is detected. A specific detection principle is as follows. First, carbon dioxide has a property of absorbing infrared rays emitted from the light emitting unit. Therefore, when the concentration of carbon dioxide flowing through the passage of the case 22 is relatively high, the ratio of the infrared rays absorbed by the carbon dioxide is correspondingly increased. Therefore, an amount of the infrared rays received by the light receiving unit becomes relatively small. Reversely, when the concentration of carbon dioxide flowing through the passage of the case 22 is relatively low, the ratio of the infrared rays absorbed by carbon dioxide is correspondingly reduced. Therefore, an amount of the infrared rays received by the light receiving unit is relatively increased. Accordingly, the concentration of carbon dioxide contained in the exhaled breath of the testee can be detected based on the amount of the infrared rays received by the light receiving unit.

In actual detection, an electric signal outputted by the light receiving unit of the sensor is sent to the measurement device through the cable 23. At this time, in the measurement device, the electric signal is converted to a numerical value (unit: mmHg) indicating the carbon dioxide concentration (EtCo₂) in the exhaled gas, by processing the electric signal given from the light receiving unit based on a predetermined algorithm. Then, the converted numerical value is displayed on the display unit of the measurement device as necessary. There is a correlation between the concentration of carbon dioxide in the exhaled breath and the concentration of carbon dioxide in arterial blood. Therefore, COPD (chronic obstructive pulmonary disease) can be accurately diagnosed by measuring the concentration of carbon dioxide in exhaled breath using the breath test device 21. Specifically, for example, when the concentration of carbon dioxide in the exhaled breath exceeds a predetermined value (for example, 45 mmHg), it is possible to make a diagnosis that COPD is strongly suspected.

### <6. Effect of the embodiment>

According to the embodiment of the present invention, the following constitution is adopted: the mouthpiece 1 is constituted by using the mouthpiece main body 2, the filter 3 and the insertion member 4, and a part of the filter 3 (the second filter portion 3b) is sandwiched between the mouthpiece main body 2 and the insertion member 4 (gap portion). Thereby, the mouthpiece 1 with a filter can be provided at a lower cost than before.

Further, the mouthpiece main body 2 has a straight tubular shape excluding the curved taper portion 9, and an entire body of the insertion member 4 also has a straight tubular shape, and therefore each component cost can be suppressed to be low. Further, although the mouthpiece 1 has a very simple constitution, it is possible to reliably hold the filter 3 in the mouthpiece main body 2.

Further, the second filter portion 3b of the filter 3 is sandwiched so as to fill the gap portion between the mouthpiece main body 2 and the insertion member 4. Therefore, the function as the filter 3 is obtained not only in the first filter portion 3a but also in the second filter portion 3b. In this case, the folded-back dimension of the second filter portion 3b is secured to be larger than the thickness dimension of the filter 3. Therefore, it is possible to effectively suppress a leakage of bacteria, virus, etc. from the gap portion between the mouthpiece main body 2 and the insertion member 4.

Further, in the mouthpiece 1 of this embodiment, the filter 3 can be held without using an adhesive agent. Therefore, there is no possibility that the user of the mouthpiece 1 feels uncomfortable with an odor of the adhesive agent or the user sucks the components of the adhesive agent. For the mouthpiece of the present invention, one of preferable modes is a configuration without using the adhesive agent, but nonuse of the adhesive agent is not an indispensable requirement.

In the embodiment of the present invention, the curved taper portion 9 is provided at one end portion of the mouthpiece main body 2. Therefore, it is possible to soften the mouth feel of the user who holds the mouthpiece 1 in his/her mouth. Further, the curved taper portion 9 can also be used as a positioning stopper for the insertion member 4 to be inserted into the mouthpiece main body 2.

In the embodiment of the present invention, as the constitution of the breath test device 21, the constitution of detachably attaching the mouthpiece 1 to the case 22 is adopted. Thereby, even when the mouthpiece 1 is handled as disposable (disposable item), the mouthpiece 1 can be provided at a low cost as described above, thus lightening a cost burden. Therefore, for example, when a breath test is performed by routine health examinations, the mouthpiece 1 can be used in place of a new one for each testee. Accordingly, the breath test can be hygienically performed without taking time to disinfect the mouthpiece 1 or the like.

### <7. Modified example etc.>

The technical scope of the present invention is not limited to the abovementioned embodiments and includes various modes and modifications within the scope of the appended claims 1-5.

For example, in the abovementioned embodiments, the cross-sectional shapes of the mouthpiece main body 2 and the insertion member 4 each having a tubular shape are circular cross sections. However, the present invention is not limited thereto, and for example, it may be a quadrangle (square, rectangle, etc.), or other polygonal shapes, elliptical shapes, combinations of straight lines and curves, or combinations of curves (arc lines) having different curvatures. However, in either case, the cross-sectional shapes of the mouthpiece main body 2 and the insertion member 4 are preferably the same shape.

Further, at least one of the mouthpiece main body 2, the filter 3 and the insertion member 4 may be made of paper.

Further, as a material of the filter 3, gauze may be used instead of a nonwoven fabric, or the nonwoven fabric and the gauze may be used in combination. Further, the filter 3 may be disposed anywhere on the flow path 6 from the opening 7 to the opening 8 of the mouthpiece main body 2.

Further, in the abovementioned embodiment, the curved taper portion 9 is provided at one end portion of the mouthpiece main body 2, thus enabling a relative positioning between the mouthpiece main body 2 and the insertion member 4 by utilizing the stopper function of the curved taper portion 9. However, the present invention is not limited thereto, and for example the following constitution may also be adopted: a diameter decreased portion in which the diameter of the mouthpiece main body 2 is partially decreased is formed in the middle of the mouthpiece main body 2 in the central axis direction, thus enabling a relative positioning between the mouthpiece main body 2 and the insertion member 4 by utilizing the stopper function of such a diameter decreased portion.

Further, according to the abovementioned embodiment, a method for manufacturing the mouthpiece 1 using the assembly jig 15 has been described as an example. However, it is also possible to manufacture the mouthpiece 1 without using the assembly jig 15. In this case, the filter 3 is put on one end portion of the insertion member 4, a portion to be the second filter portion 3b is folded-back, and in this state, the insertion member 4 may be inserted (press-fitted) into the mouthpiece main body 2. However, in order to manufacture the mouthpiece 1 efficiently with stable quality, it is desirable to use the abovementioned assembly jig 15 or another assembly jig instead.

Further, according to the abovementioned embodiment, explanation is given for the following case as an example: the concentration of carbon dioxide contained in the exhaled breath is tested (detected) by using the breath test device 21. However, the present invention is not limited thereto, and is also applicable to, for example, the case of inspecting oxygen concentration, nitric oxide concentration, alcohol concentration, and the like, contained in exhaled breath.

Further, the mouthpiece of the present invention is not limited to the case of a use by being attached to the breath test device, and it is possible to widely use the mouthpiece in various situations such as breathing in or out, or breathing in and out, in a state that the mouthpiece is held in the mouth. Specifically, for example, the mouthpiece of the present invention can be used by being attached to a pulmonary function measurement device, a ventilator or the like. In this case, dimensions (outer diameter, length, etc.) of the mouthpiece may be set in accordance with each use and equipment.

### Description of signs and numerals

- 1: Mouthpiece
- 2: Mouthpiece main body
- 3: Filter
- 3a: First filter portion
- 3b: Second filter portion
- 4: Insertion member
- 6: Flow path
- 9: Curved taper portion
- 21: Breath test device

## Claims

1. A mouthpiece (1), comprising:
a tubular mouthpiece main body (2) configured to form a flow path (6) for flowing a breathing gas;
a filter (3) placed on the flow path (6); and
a tubular insertion member (4) inserted into the mouthpiece main body (2) in order to hold the filter (3) in the inside of the mouthpiece main body (2) and having a length not to protrude from the mouthpiece main body (2),
wherein the filter (3) has a first filter portion (3a) corresponding to a cross-sectional shape of the flow path (6) and a second filter portion (3b) folded-back from an outer circumferential edge of the first filter portion (3a), and the second filter portion (3b) is sandwiched between an inner circumferential surface of the mouthpiece main body (2) and an outer circumferential surface of the insertion member (4).

2. The mouthpiece (1) according to claim 1, wherein a folded-back dimension of the second filter portion (3b) is 2 mm or more.

3. The mouthpiece (1) according to claim 1 or 2, wherein a curved taper portion (9) is provided at one end portion of the mouthpiece main body (2).

4. A breath test device (21), comprising:
the mouthpiece (1) of any one of claims 1 to 3, and
a member (22) to which the mouthpiece (1) is detachably attached,
wherein a predetermined component contained in an exhaled breath supplied through the mouthpiece (1) is tested.

5. A method for manufacturing a mouthpiece (1), comprising:
preparing a tubular mouthpiece main body (2) configured to form a flow path (6) for flowing a breathing gas, a filter (3) placed on the flow path (6), and a tubular insertion member (4) inserted into the mouthpiece main body (2) in order to hold the filter (3) in the inside of the mouthpiece main body (2) and having a length not to protrude from the mouthpiece main body (2); and
forming a first filter portion (3a) corresponding to a cross-sectional shape of the flow path (6) by covering one end portion of the insertion member (4) with the filter (3), and
sandwiching a second filter portion (3a) being a folded-back portion of the filter (3) between an inner circumferential surface of the mouthpiece main body (2) and an outer circumferential surface of the insertion member (4), by folding-back a part of the filter (3) and inserting the insertion member (4) into the mouthpiece main body (2).

## Patentansprüche

1. Mundstück (1), umfassend:
einen rohrförmigen Mundstückhauptkörper (2), der dazu konfiguriert ist, einen Strömungspfad (6) für eine Strömung eines Atemgases zu bilden;
einen Filter (3), der an dem Strömungspfad (6) platziert ist; und
ein rohrförmiges Einsetzelement (4), das in dem Mundstückhauptkörper (2) eingesetzt ist, um den Filter (3) im Inneren des Mundstückhauptkörpers (2) zu halten, und das eine Länge derart hat, dass es nicht von dem Mundstückhauptkörper (2) vorsteht,
wobei der Filter (3) einen ersten Filterbereich (3a) hat, der einer Querschnittsgestalt des Strömungspfads (6) entspricht, und einen zweiten Filterbereich (3b), der von einem äußeren Umfangsrand des ersten Filterbereichs (3a) zurückgefaltet ist, und wobei der zweite Filterbereich (3b) sandwichförmig zwischen einer inneren Umfangsoberfläche des Mundstückhauptkörpers (2) und einer äußeren Umfangsoberfläche des Einsetzelements (4) eingefügt ist.

2. Mundstück (1) nach Anspruch 1, wobei eine zurückgefaltete Abmessung des zweiten Filterbereichs (3b) zwei mm oder mehr ist.

3. Mundstück (1) nach Anspruch 1 oder 2, wobei ein gekrümmter Verjüngungsbereich (9) an einem Endbereich des Mundstückhauptkörpers (2) vorgesehen ist.

4. Atemtestvorrichtung (21), umfassend:
das Mundstück (1) nach einem der Ansprüche 1 bis 3, und
ein Element (22), an dem das Mundstück (1) lösbar angebracht ist, wobei eine vorbestimmte Komponente getestet wird, die in einem ausgestossenen Atem enthalten ist, der durch das Mundstück (1) bereitgestellt wird.

5. Verfahren zur Herstellung eines Mundstücks (1), umfassend:
Herstellen eines rohrförmigen Mundstückhauptkörpers (2), der dazu konfiguriert ist, einen Strömungspfad (6) für eine Strömung eines Atemgases zu bilden, eines Filters (3), der an dem Strömungspfad (6) platziert ist, und eines rohrförmigen Einsetzelements (4), das in den Mundstückhauptkörper (2) eingesetzt wird, um den Filter (3) im Inneren des Mundstückhauptkörpers (2) zu halten, und das eine Länge derart hat, dass es nicht von dem Mundstückhauptkörper (2) vorsteht; und
Bilden eines ersten Filterbereichs (3a), der einer Querschnittsgestalt des Strömungspfads (6) entspricht, durch Bedecken eines Endbereichs des Einsetzelements (4) mit dem Filter (3), und
sandwichförmiges Einfügen eines zweiten Filterbereichs (3a), der ein zurückgefalteter Bereich des Filters (3) ist, zwischen einer inneren Umfangsoberfläche des Mundstückhauptkörpers (2) und einer äußeren Umfangsoberfläche des Einsetzelements (4) durch Zurückfalten eines Teils des Filters (3) und Einsetzen des Einsetzelements (4) in den Mundstückhauptkörper (2).

## Revendications

1. Embout buccal (1) comprenant :
un corps principal d'embout buccal tubulaire (2) configuré pour former une trajectoire d'écoulement (6) pour l'écoulement d'un gaz respiratoire ;
un filtre (3) placé sur la trajectoire d'écoulement (6) ; et
un élément d'insertion tubulaire (4) inséré dans le corps principal d'embout buccal (2) afin de maintenir le filtre (3) à l'intérieur du corps principal d'embout buccal (2) et ayant une longueur ne faisant pas saillie du corps principal d'embout buccal (2),
dans lequel le filtre (3) a une première partie de filtre (3a) correspondant à une forme transversale de la trajectoire d'écoulement (6) et une seconde partie de filtre (3b) repliée à partir d'un bord circonférentiel externe de la première partie de filtre (3a), et la seconde partie de filtre (3b) est prise en sandwich entre une surface circonférentielle interne du corps principal d'embout buccal (2) et une surface circonférentielle externe de l'élément d'insertion (4).

2. Embout buccal (1) selon la revendication 1, dans lequel une dimension repliée de la seconde partie de filtre (3b) est de 2 mm ou plus.

3. Embout buccal (1) selon la revendication 1 ou 2, dans lequel une partie de conicité incurvée (9) est prévue au niveau d'une partie d'extrémité du corps principal d'embout buccal (2).

4. Dispositif de test respiratoire (21) comprenant :
l'embout buccal (1) selon l'une quelconque des revendications 1 à 3, et
un élément (22) auquel l'embout buccal (1) est fixé de manière détachable,
dans lequel un composant prédéterminé contenu dans une respiration exhalée amenée par l'embout buccal (1) est testé.

5. Procédé pour fabriquer un embout buccal (1) comprenant les étapes consistant à :
préparer un corps principal d'embout buccal tubulaire (2) configuré pour former une trajectoire d'écoulement (6) pour l'écoulement d'un gaz respiratoire, un filtre (3) placé sur la trajectoire d'écoulement (6), et un élément d'insertion tubulaire (4) inséré dans le corps principal d'embout buccal (2) afin de maintenir le filtre (3) à l'intérieur du corps principal d'embout buccal (2) et ayant une longueur ne faisant pas saillie du corps principal d'embout buccal (2) ; et
former une première partie de filtre (3a) correspondant à une forme transversale de la trajectoire d'écoulement (6) en recouvrant une partie d'extrémité de l'élément d'insertion (4) avec le filtre (3), et
prendre en sandwich une seconde partie de filtre (3a) qui est une partie repliée du filtre (3) entre une surface circonférentielle interne du corps principal d'embout buccal (2) et une surface circonférentielle externe de l'élément d'insertion (4), en repliant une partie du filtre (3) et en insérant l'élément d'insertion (4) dans le corps d'embout buccal (2).
